# EUROPEAN PATENT APPLICATION

(11) **EP 4 717 306 A1**
(43) Date of publication of application: **01.04.2026**
(21) Application number: 25204576.0
(22) Date of filing: 25.09.2025
(51) Int. Cl.: A61N 5/10

(54) **IN-SYSTEM GENERATION OF QUALITY ASSURANCE BEAM PLANS FOR QUALITY ASSURANCE TESTING OF A RADIATION THERAPY SYSTEM**

(30) Priority: 27.09.2024 US 202463700522 P; 12.11.2024 US 202418943926
(71) Applicant: Varian Medical Systems, Inc., Palo Alto, CA 94304 (US)
(72) Inventor: HOLMES, Todd, Bethlehem, 18017 (US); MÜLLER, Sebastijan, 5507 Mellingen (CH); PFLUGI, Silvio, 5033 Buchs AG (CH)
(74) Representative: Mathisen & Macara LLP

(57) **Abstract**

A computer-implemented method of performing a quality assurance (QA) test on a treatment delivery system is disclosed. The method includes generating 632 a beam plan template 561 for performing the QA test on the treatment delivery system 300, wherein the beam plan template is based on a testing guideline for the QA test, in response to a first user input, determining 731, with the one or more processors, one or more system-specific capabilities of the treatment delivery system at the time of receipt of the first user input, generating 732 a QA beam plan for performing the QA test on the treatment delivery system, wherein the QA beam plan is based on the beam plan template and the one or more system-specific capabilities of the treatment delivery system, and causing the treatment delivery system to perform 751 the QA beam plan.

## Description

### CROSS-REFERENCE TO RELATED APPLICATION

The present application claims the benefit of U.S. Provisional Application No. 63/700,522, filed September 27, 2024. The aforementioned U.S. Provisional Application, including any appendices or attachments thereof, is hereby incorporated by reference in its entirety.

### BACKGROUND

Unless otherwise indicated herein, the approaches described in this section are not prior art to the claims in this application and are not admitted to be prior art by inclusion in this section.

Radiation therapy (also called radiotherapy) is a cancer treatment that employs high doses of ionizing radiation, such as X-rays or high-energy electrons, protons, or other heavy charged particles, to kill cancer cells. Generally, radiation therapy is a localized treatment for a specific target tissue, such as a cancerous tumor. Ideally, radiation therapy is performed on a planning target volume (i.e., the target tissue) that spares the surrounding normal tissue from receiving doses above specified tolerances, thereby minimizing risk of damage to healthy tissue. For example, to accurately supply a planned radiation dose, the spatial distribution of delivered radiation dose within the patient must closely match the spatial distribution of the planned radiation dose. So that the planned radiation dose is correctly supplied to the planning target volume during radiation therapy, the patient should be correctly positioned relative to the radiation source that provides the radiation therapy. In addition, precisely controlling the position of the radiation source relative to the patient is a significant factor in accurately targeting tissue in the patient. Furthermore, to detect and/or compensate for patient motion during a particular radiation therapy session, or "fraction," patient motion is often monitored in near-real time using optical and/or X-ray imaging techniques.

To ensure that the complex drive systems, imaging systems, and radiation sources of radiation therapy systems operate with suitable accuracy, precision, and repeatability, numerous quality assurance testing protocols have been developed. These testing protocols include structured procedures and actions that can maintain a high level of treatment quality of patients and prevent accidents during a radiation therapy session.

### SUMMARY

According to various embodiments, a treatment delivery system can generate a beam plan for performing a specific quality assurance (QA) test. In the embodiments, a QA application running locally on the treatment delivery system generates a beam plan template for performing the specific QA test on the treatment delivery system. The beam plan template includes the set of predefined motions and operations that are executed by the treatment delivery system in order to perform the QA test. The QA application can generate the beam plan template based on a publicly known testing guideline for the specific QA test. As a result, a user is not required to provide detailed inputs for a beam plan template to be generated for a specific QA test. Further, in the embodiments, the QA application generates a QA beam plan for performing the specific QA test on the treatment delivery system at the time of the QA test, where the QA beam plan includes machine-readable commands for causing the treatment delivery system to perform the QA test. Specifically, the QA application generates the QA beam plan for a specific QA test based on the beam plan template for that QA test and on one or more system-specific capabilities of the treatment delivery system as currently configured. Consequently, a user is not required to construct the QA beam plan by entering system-specific capabilities of the treatment delivery system into a treatment planning system that is separate from the treatment delivery system. Instead, the QA beam plan is generated locally by the QA application using the most up-to-date capabilities and settings of the treatment delivery system, which are detected by the locally running QA application. As a result, a QA beam plan for performing a QA test on a treatment delivery system can be generated locally on the treatment delivery system with minimal user inputs and with accurate capability and setting information for the treatment delivery system.

According to some embodiments, a computer-implemented method of performing a QA test on a treatment delivery system includes: with one or more processors of the treatment delivery system, generating a beam plan template for performing the QA test on the treatment delivery system, wherein the beam plan template is based on a testing guideline for the QA test; in response to a first user input, determining, with the one or more processors, one or more system-specific capabilities of the treatment delivery system at the time of receipt of the first user input; generating, with the one or more processors, a QA beam plan for performing the QA test on the treatment delivery system, wherein the QA beam plan is based on the beam plan template and the one or more system-specific capabilities of the treatment delivery system; and causing, with the one or more processors, the treatment delivery system to perform the QA beam plan.

According to some embodiments, a treatment delivery system includes: a radiation source mounted on a gantry that rotationally positions the radiation source about an isocenter of the treatment delivery system; and a controller. In the embodiments, the controller is configured to perform the steps of: generating a beam plan template for performing a QA test on the treatment delivery system, wherein the beam plan template is based on a testing guideline for the QA test; in response to a first user input, determining one or more system-specific capabilities of the treatment delivery system at the time of receipt of the first user input; generating a QA beam plan for performing the QA test on the treatment delivery system, wherein the QA beam plan is based on the beam plan template and the one or more system-specific capabilities of the treatment delivery system; and causing the treatment delivery system to perform the QA beam plan.

Further embodiments include a non-transitory computer-readable storage medium comprising instructions that cause a computer system to carry out one or more of the above methods, as well as a computer system configured to carry out one or more of the above methods.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

The foregoing and other features of the present disclosure will become more fully apparent from the following description and appended claims, taken in conjunction with the accompanying drawings. These drawings depict only several embodiments in accordance with the disclosure and are, therefore, not to be considered limiting of its scope. The disclosure will be described with additional specificity and detail through use of the accompanying drawings.
FIG. 1 is a perspective view of a radiation therapy system that can beneficially implement various embodiments.
FIG. 2 schematically illustrates a side view of the radiation therapy system of FIG. 1, according to various embodiments.
FIG. 3 is a perspective view of a radiation therapy system, according to various embodiments.
FIG. 4 schematically illustrates a base stand and a gantry of the radiation therapy system of FIG. 3, according to various embodiments.
FIG. 5 conceptually illustrates a QA application incorporated in a control system for a treatment delivery system, according to various embodiments
FIG. 6 is a flowchart illustrating the steps performed by the QA application of FIG. 5 and a user interacting with the QA application as part of a QA task planning process, according to various embodiments.
FIG. 7 is a flowchart illustrating the steps performed by the QA application of FIG. 5 and a user interacting with the QA application as part of a QA process, according to various embodiments.
FIG. 8 is an illustration of a computing device configured to perform various embodiments of the present disclosure.
FIG. 9 is a block diagram of an illustrative embodiment of a computer program product 900 for implementing a various methods described herein, according to various embodiments.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here. It will be readily understood that the aspects of the disclosure, as generally described herein, and illustrated in the figures, can be arranged, substituted, combined, and designed in a wide variety of different configurations, all of which are explicitly contemplated and make part of this disclosure. Although the terms "first" and "second" are used to describe various elements, these elements should not be limited by these terms. These terms are used to distinguish one element from another. For example, a first element may be referred to as a second element, and vice versa. Independent of the grammatical term usage, individuals with male, female or other gender identities are included within the term.

### Introduction

As noted previously, to maintain the stringent requirements of radiation therapy treatment, various quality assurance (QA) testing protocols have been developed for radiation therapy systems. These QA testing protocols ensure that the complex drive systems, imaging systems, and radiation sources of a particular radiation therapy system operate with suitable accuracy, precision, and repeatability. Typically, a particular QA testing protocol is performed on a radiation therapy system at regular intervals (e.g., daily, weekly, monthly, annually), and includes a specific series of operations that are performed by the system. For example, in a QA test that confirms mechanical alignment of the radiation therapy system, some or all geometric axes of the system (e.g., gantry angle, collimator angle, field size, couch position, etc.) are driven to specified positions. In another example, in a QA test that quantifies image quality, certain image acquisition parameters (e.g., imaging mode, imaging source voltage, imaging source power, etc.) are set to target values and an X-ray image is then acquired. In yet other examples, a QA test may include a combination of mechanical and beam-related checks. There are various conventional approaches a QA performer (such as a radiation therapist or medical physicist) can employ for performing a QA test, but each has one or more drawbacks.

In some instances, the QA performer performs a particular QA test by manually running the radiation therapy system, for example while the system is in a service mode. This entails manual positioning of all pertinent geometric axes, manually programming specific values for one or more beam parameters (e.g., beam energy, monitor units, dose rate, etc.) and/or image acquisition parameters, and then manually executing a "beam on" command. Thus, the QA performer is required to manually control various components and subsytems of the radiation therapy system for each operation included in the QA test. This approach is time-consuming, can require specialized training and extensive knowledge of the radiation therapy system, and is prone to error.

In some instances, the QA performer performs a particular QA test by executing a treatment plan that has been generated on a separate treatment planning system and is tailored to cause the operations for that particular QA test to be performed by a particular radiation therapy system. With this approach, most of the QA process is automated, and the actions of the QA performer are simplified to selecting the appropriate treatment plan at the time of the QA test. As a result, the use of a treatment plan to execute a QA test greatly streamlines the QA process. However, such treatment plans must be generated in a computerized treatment planning system that is normally used to generate beam shapes and dose distributions for a variety of different radiation therapy systems. To maximize tumor control and minimize normal tissue complications, the treatment planning process involves many steps, including beam data and patient data acquisition, entry of the beam data and patient data into the treatment planning system, treatment plan generation, and distribution of the treatment plan to the correct radiation treatment system. Consequently, treatment planning systems require specialized training to operate, even when generating the relatively simple treatment plans employed for QA testing. Thus, only certain specialists can generate a treatment plan via a treatment planning system. An additional drawback of treatment planning systems is that, as a separate computerized system from the treatment system that undergoes the QA test, system-specific information must be provided to the treatment planning system for a suitable treatment to be generated. Because radiation therapy systems are complex and can operate using various settings or capabilities that change over time, providing completely accurate and up-to-date system-specific information to the treatment planning system can be time-consuming and a potential source of error when a treatment plan is generated. Furthermore, once a treatment plan is generated for a QA test of a particular radiation therapy system, the treatment plan cannot be implemented by the radiation therapy system unless correctly cataloged and imported to the radiation treatment system. In some instances, once the treatment plan for a QA test is imported to the radiation therapy system, additional measurements need to be taken to confirm that the system capabilities assumed by the treatment planning system actually match the system capabilities of the radiation treatment system. In some instances, the QA test treatment plan must also be added to a treatment schedule before the radiation therapy system can execute the treatment plan. Thus, the use of a separate treatment planning system to generate a QA test treatment plan can be problematic for a number of reasons.

In some instances, the QA performer performs a particular QA test by executing a QA treatment plan that is provided with the radiation therapy system. For example, a manufacturer of the radiation therapy system may generate such QA treatment plans for certain predefined QA tests and incorporate these treatment plans into the control software of the radiation therapy system. One drawback of this approach is that the capabilities of a particular radiation therapy system can change over time, for example due to the removal or replacement of certain hardware, the modification of system software, or the updating of licensed features on the radiation therapy system. Once such changes occur, one or more of the existing QA treatment plans available on the radiation therapy system can become obsolete or otherwise not applicable for the intended QA test. Another drawback of this approach is that manufacturer-provided QA treatment plans are for generic QA tests. Thus, when a QA test is required that is based on a site-specific use case for a particular radiation therapy system, a user of that particular radiation therapy system must generate the QA treatment plan in some other way, for example via a treatment planning system.

Accordingly, there is a need in the art for improved methods for performing QA testing of a radiation therapy system.

### System Overview

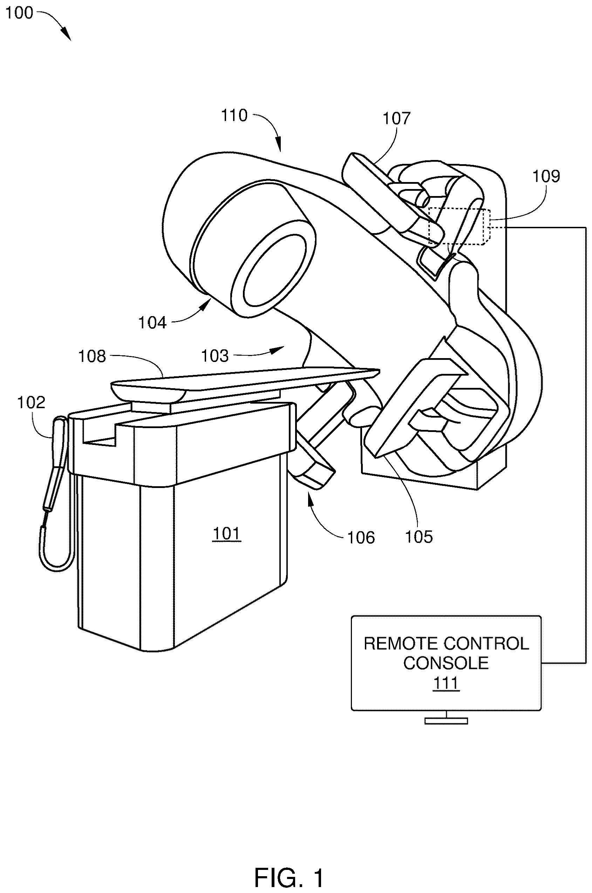

FIG. 1 is a perspective view of a radiation therapy system 100 that can beneficially implement various embodiments. Radiation therapy (RT) system 100 is a radiation system that may be configured to detect intra-fraction motion in near-real time using either optical or X-ray imaging techniques, or both. Thus, in some embodiments, RT system 100 is configured to provide stereotactic radiosurgery and precision radiotherapy for lesions, tumors, and conditions anywhere in the body where radiation treatment is indicated. As such, RT system 100 can include one or more of a linear accelerator (LINAC) 104 that generates an MV treatment beam of high energy X-rays or other radiation, one or more kilovolt (kV) imaging X-ray sources 106, one or more imaging panels 107 (e.g., an X-ray imager), and a mega-Volt (MV) electronic portal imaging device (EPID) 105. In the embodiment illustrated in FIG. 1, RT system 100 is configured with a C-arm gantry 110 capable of infinite rotation via a slip ring connection.

In some embodiments, RT system 100 is capable of X-ray imaging of a target volume immediately prior to and/or during application of an MV treatment beam, so that an image-guided radiation therapy (IGRT) and/or an intensity-modulated radiation therapy (IMRT) process can be performed using X-ray imaging. For example, in some embodiments, such processes can include kV imaging of the target volume in conjunction with imaging generated by the MV treatment beam. RT system 100 may include one or more touchscreens (not shown) for patient information verification, couch motion controls 102, a radiation area 103, a couch positioning assembly 101, a couch 108 disposed on couch positioning assembly 101, and an image acquisition and treatment control computer 109, all of which are disposed within a treatment room. RT system 100 further includes a remote control console 111, which is disposed outside the treatment room and enables treatment delivery and patient monitoring from a remote location. Couch positioning assembly 101 is configured to precisely position couch 108 with respect to radiation area 103. Motion controls 102 include input devices, such as buttons and/or switches, that enable a user to operate couch positioning assembly 101 to automatically and precisely position couch 108 to a predetermined location with respect to radiation area 103. Motion controls 102 also enable a user to manually position couch 108 to a particular location, such as a planned treatment position for a patient or anatomical target.

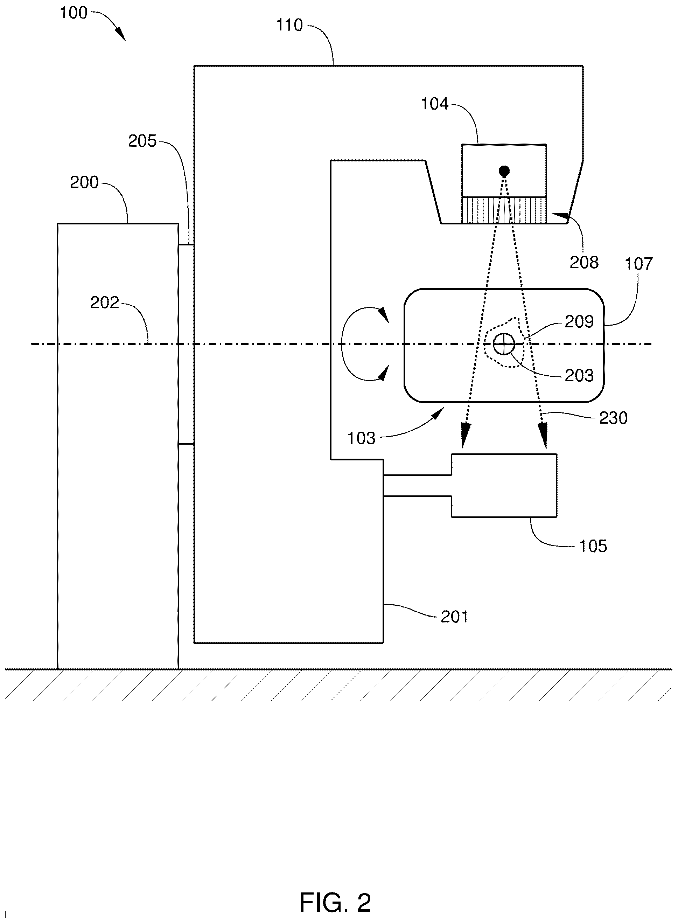

FIG. 2 schematically illustrates a side view of RT system 100, according to various embodiments. As shown, RT system 100 includes a base stand 200 and C-arm gantry 110. In FIG. 2, couch positioning assembly 101, couch 108, and imaging X-ray source 106 are omitted for clarity. Base stand 200 is a fixed support structure for components of RT treatment system 100, including C-arm gantry 110 and a drive system (not shown) for rotatably moving C-arm gantry 110 about a horizontal rotation axis 202. Base stand 200 rests on and/or is fixed to a support surface that is external to RT treatment system 100, such as a floor of an RT treatment facility. C-arm gantry 110 is rotationally coupled to base stand 200, for example via a bearing 205 (cross-hatched). C-arm gantry 110 is a support structure on which various components of RT system 100 are mounted, including LINAC 104, EPID 105, imaging X-ray source 106 (not shown in FIG. 2 for clarity), and imaging panel 107.

In the embodiment illustrated in FIG. 2, imaging panel 107 is depicted as a planar device, whereas in other embodiments, imaging panel 107 can have a curved configuration. In the embodiment illustrated in FIGS. 1 and 2, RT system 100 includes a single imaging panel and a single corresponding imaging radiation source in addition to EPID 105. In other embodiments, RT system 100 can include two or more imaging panels, each with a corresponding imaging radiation source. Further, in some embodiments, couch positioning assembly 101 is configured to rotate, pitch, roll, and/or translate couch 108 sequentially relative to isocenter 203 to one or more treatment positions.

LINAC 104 is a radiation source, and typically includes one or more of an electron gun for generating electrons, an accelerating waveguide, an electron beam target, an electron beam transport means (such as a bending magnet) for directing the electron beam to the electron beam target, and/or a collimator assembly 208 for collimating and shaping a treatment beam 230 that originates from the electron beam target. Collimator assembly 208 typically includes one or more of a primary collimator that defines the largest available circular radiation field for treatment beam 230, a secondary collimator for providing a rectangular or square radiation field at isocenter 203 (for example via X-jaws and Y-jaws), and a multileaf collimator (MLC) for conforming treatment beam 230 to a planning target volume (PTV) or other anatomical target. In other embodiments, LINAC 104 can be any other radiation source suitable for radiation therapy.

During radiation treatment, LINAC 104 is configured to generate treatment beam 230, which can include high-energy radiation (for example MV X-rays or MV electrons). In other embodiments, treatment beam 230 includes electrons, protons, and/or other heavy charged particles, ultra-high dose rate X-rays (e.g., for FLASH radiotherapy), and/or microbeams for microbeam radiation therapy. In addition, imaging panel 107 is configured to receive imaging radiation and generate suitable projection images therefrom. Further, in some embodiments, as treatment beam 230 is directed to isocenter 203 while C-arm gantry 110 rotates through a treatment arc, image acquisitions can be performed via EPID 105 to generate image data for target volume 209. For example, in such embodiments, EPID 105 generates one or more projection images of target volume 209 and/or a region of patient anatomy surrounding target volume 209. Thus, projection images (e.g., 2D X-ray images) of target volume 209 can be generated during portions of an IGRT or IMRT process via imaging panel 107 and/or EPID 105. Such projection images can then be employed to construct or update portions of imaging data for a digital volume that corresponds to a three-dimensional (3D) region that includes target volume 209. That is, a 3D image of such a 3D region is reconstructed from the projection images. In some embodiments, cone-beam computed tomography (CBCT) and/or digital tomosynthesis (DTS) can be used to process the projection images generated by imaging panel 107.

During operation of RT treatment system 100, C-arm gantry 110 rotates about radiation area 103 when actuated by the drive system for rotatably moving C-arm gantry 110 about horizontal rotation axis 202. Imaging X-ray source 106 is configured to direct a conical beam of X-rays, referred to herein as imaging X-rays (not shown in FIG. 2 for clarity), through an isocenter 203 of RT system 100 to imaging panel 107. Ideally, isocenter 203 corresponds to the location of a target volume 209 to be treated, such as a PTV, a gross tumor volume (GTV), a clinical target volume (CTV), and/or an internal target volume (ITV), among others. According to various embodiments, a drive system of RT treatment system 100 enables precise and repeatable rotational positioning of LINAC 104 (or any other suitable radiation source) about isocenter 203. Various embodiments of such a drive system are described below.

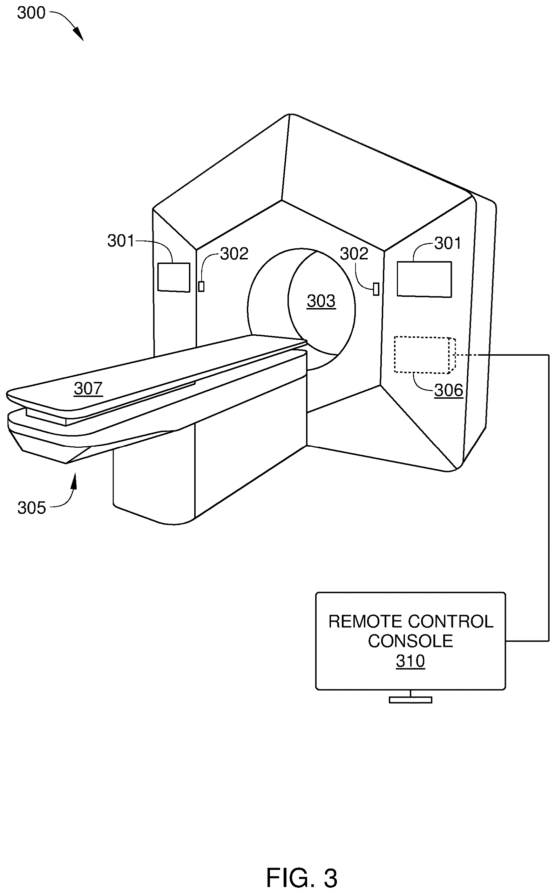

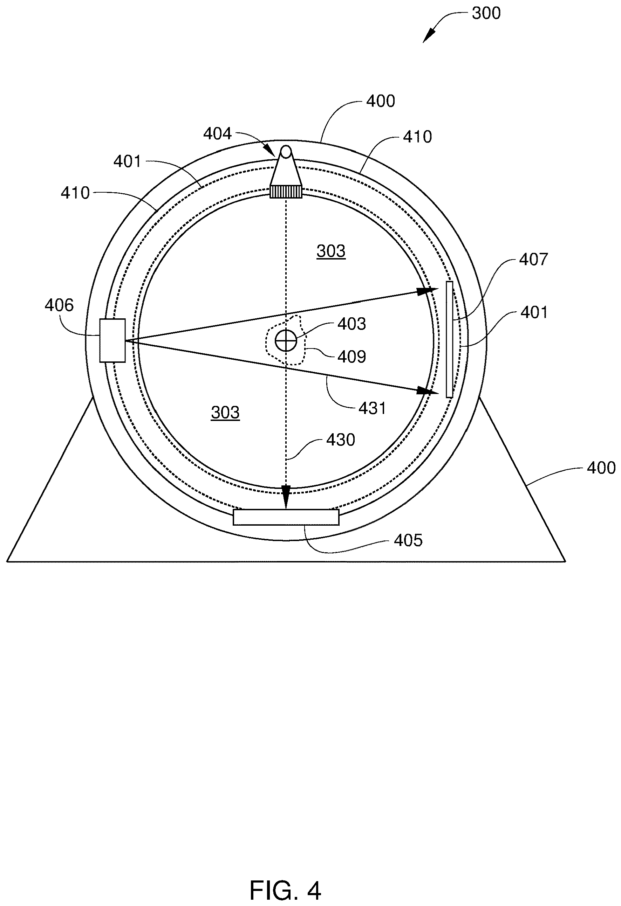

In the embodiment illustrated in FIGS. 1 and 2, C-arm gantry 110 of RT system 100 is capable of infinite rotation via a slip ring connection. In other embodiments, a radiation therapy system can be configured with a ring-based gantry that is disposed about an isocenter of the radiation therapy system. One such embodiment is described below in conjunction with FIGS. 3 and 4.

FIG. 3 is a perspective view of an RT system 300, according to various embodiments. In some embodiments, RT system 300 can be consistent with RT system 100 of FIGS. 1 and 2. In FIG. 3 RT system 300 is configured with a circular or ring-based gantry. As such, RT system 300 can include one or more touchscreens 301, couch motion controls 302, a bore 303, a base positioning assembly 305, a couch 307 disposed on base positioning assembly 305, and an image acquisition and treatment control computer 306, all of which are disposed within a treatment room. RT system 300 further includes a remote control console 310, which is disposed outside the treatment room and enables treatment delivery and patient monitoring from a remote location. Base positioning assembly 305 is configured to precisely position couch 307 with respect to bore 303, and motion controls 302 include input devices, such as button and/or switches, that enable a user to operate base positioning assembly 305 to automatically and precisely position couch 307 to a predetermined location with respect to bore 303. Motion controls 302 also enable a user to manually position couch 307 to a predetermined location.

FIG. 4 schematically illustrates a base stand 400 and gantry 410 of RT system 300, according to various embodiments. Covers, base positioning assembly 305, couch 307, and other components of RT system 300 are omitted in FIG. 4 for clarity. Base stand 400 is a fixed support structure for components of RT system 300, including gantry 410 and a drive system 401 (dashed lines) for rotatably moving gantry 410. Base stand 400 rests on and/or is fixed to a support surface that is external to RT system 300, such as a floor of a radiotherapy treatment facility. Gantry 410 is rotationally coupled to base stand 400 and is a support structure on which various components of RT system 300 are mounted, including a LINAC 404, an EPID 405, an imaging X-ray source 406, and an X-ray imager 407.

During operation of RT system 300, drive system 401 rotationally actuates gantry 410, so that gantry 410 rotates about bore 303. LINAC 404 generates an MV treatment beam 430 of high energy X-rays (or in some embodiments electrons, protons, and/or other heavy charged particles, ultra-high dose rate X-rays (e.g., for FLASH radiotherapy) or microbeams for microbeam radiation therapy) and EPID 405 is configured to acquire X-ray images with treatment beam 430. Imaging X-ray source 406 is configured to direct a conical beam of X-rays, referred to herein as imaging X-rays 431, through an isocenter 403 of RT system 300 to X-ray imager 407, and isocenter 403 typically corresponds to the location of a target volume 409 to be treated. In the embodiment illustrated in FIG. 4, X-ray imager 407 is depicted as a planar device, whereas in other embodiments, X-ray imager 407 can have a curved configuration. In the embodiment illustrated in FIG. 4, RT system 300 includes a single X-ray imager and a single corresponding imaging X-ray source. In other embodiments, RT system 300 can include two or more X-ray imagers, each with a corresponding imaging X-ray source.

### QA Application for Generating QA Beam Plan Locally on Treatment Delivery System

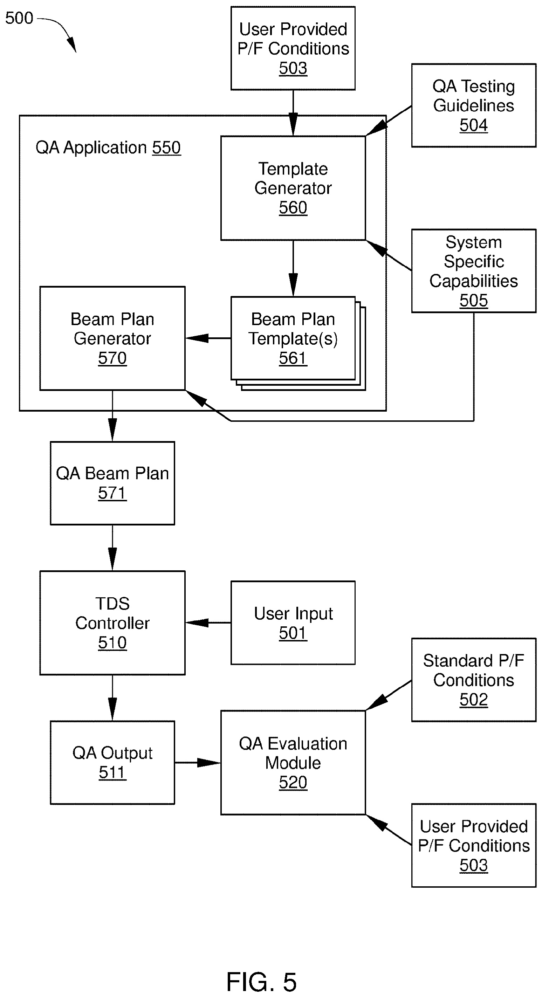

According to various embodiments, a QA application running locally on a treatment delivery system, such as RT system 100 or RT system 300, generates a beam plan template for performing a specific QA test on the treatment delivery system. Generally, the beam plan template is based on a publicly known testing guideline for the specific QA test and indicates the motions and other operations of the treatment delivery system during execution of that specific QA test. At the time of running the specific QA test, the QA application generates a QA beam plan for performing the specific QA test on the treatment delivery system based on the beam plan template for that QA test and on one or more system-specific capabilities of the treatment delivery system as currently configured. One embodiment of such a QA application is described below in conjunction with FIG. 5.

FIG. 5 conceptually illustrates a QA application 550 incorporated in a control system 500 for a treatment delivery system, according to various embodiments. Control system 500 can be a control system for a treatment delivery system for radiation therapy, such as RT system 100 of FIGS. 1 and 2 or RT system 300 of FIGS. 3 and 4. Thus, in some embodiments, control system 500 can be implemented as image acquisition and treatment control computer 109 of RT system 100 or image acquisition and treatment control computer 306 of RT system 300. As shown, control system 500 includes a treatment delivery system (TDS) controller 510, a QA evaluation module 520, and QA application 550. In the embodiment illustrated in FIG. 5, QA evaluation module 520 is implemented as a separate entity from QA application 550. In alternative embodiments, QA evaluation module 520 can be implemented as a part of QA application 550. Alternatively, in some embodiments, QA evaluation module 520 can reside and/or run outside of control system 500. In such embodiments, QA evaluation module can be associated with a computing system separate from the treatment delivery system associated with control system 500.

TDS controller 510 controls the operation of an associated treatment delivery system (referred to hereinafter as "the TDS"), such as RT system 100 or RT system 300. Generally, TDS controller 510 controls the operation of the various mechanical systems, imaging systems, and radiation delivery systems of the TDS. In operation, TDS controller 510 causes the TDS to perform a treatment fraction based on a treatment plan (not shown) and one or more user inputs 501. User inputs can include inputs made, for example via remote control console 111 in FIG. 1 or remote control console 310 in FIG. 3. Similarly, when the TDS is in a QA mode, TDS controller 510 causes the TDS to perform a QA test based on a QA beam plan 571 and user inputs 501.

In some embodiments, QA beam plan 571 includes machine-readable commands for causing the TDS to perform the operations of a QA test, including positioning of all geometric axes that are active during the QA test (e.g., gantry angle, collimator angle, field size, couch position, and the like), programming of treatment beam parameters (e.g., beam energy, monitor units, dose rate, and the like) when the treatment beam is employed in the QA test, and/or image acquisition parameters (imaging mode, kV, mAs, etc.) when an imaging beam is employed in the QA test. In some embodiments, QA beam plan 571 has a file format that includes human-readable text. For example, in some embodiments, QA beam plan 571 is implemented as an Extensible Markup Language (XML) file, a JavaScript Object Notation (JSON) file, or a Digital Imaging and Communications in Medicine (DICOM) file. In other embodiments, QA beam plan 571 can have any other suitable file format that can be employed by TDS controller 510 to control the operations of the TDS during a QA test.

During execution of and/or upon completion of QA beam plan 571, a QA output 511 is generated that is appropriate for the QA test associated with execution of QA beam plan 571. In some embodiments, QA output 511 is generated by TDS controller 510 and/or the TDS, such as one or more X-ray images, trajectory log files that report mechanical positions read by encoders included in the TDS, and the like. Alternatively or additionally, in some embodiments, QA output 511 can be generated by a user, such as a manual measurement that is performed by the user during execution of or upon completion of QA beam plan 571. For example, in such embodiments, QA output 511 can include a physical confirmation of a gantry position, a measurement of a light field size, and/or a confirmation of a safety feature operating correctly (e.g., door interlock functions, audio system captures audio from treatment room, video system captures video of treatment room or couch, and the like).

QA evaluation module 520 determines whether the TDS has passed or failed the specific QA test associated with execution of QA beam plan 571. In the embodiment illustrated in FIG. 5, QA evaluation module 520 makes the pass/fail determination based on QA output 511 and certain pass/fail (P/F) conditions, which may include standard P/F conditions 502 and/or user provided P/F conditions 503. In some embodiments, standard P/F conditions 502 can include values provided by a publicly known testing guideline. For example, in such embodiments, standard P/F conditions 502 can include values provided in guidelines published by the American Association of Physicists in Medicine (AAPM). Alternatively or additionally, in some embodiments, standard P/F conditions 502 can include values provided by the manufacturer of the TDS, such as when the TDS manufacturer creates a new capability for which there are no published guidelines. User provided P/F conditions 503 can include values input by a user of the TDS. For example, in some embodiments, user provided P/F conditions 503 are input by a user when QA application 550 generates the beam plan template 571 used to generate QA beam plan 571. In such embodiments, user provided P/F conditions 503 enable a specific TDS to be tested according to values for pass/fail parameters that are selected by a user of that specific TDS. In this way, user-specified pass/fail conditions can be implemented for a particular QA test without the need for a user to generate an entire QA beam plan manually or via a separate treatment planning system.

In operation, QA evaluation module 520 reads and analyzes images, such as X-ray images or beam-spot images, and/or other measurements taken during the execution of QA beam plan 571. QA evaluation module 520 then compares the images and/or other measurements to standard P/F conditions 502 and/or user provided P/F conditions 503 as appropriate, and notifies the user that the TDS has passed or failed the QA test associated with QA beam plan 571. For example, in one embodiment, when the QA test associated with execution of QA beam plan 571 includes acquiring a beam spot image of a treatment beam, QA evaluation module 520 analyzes the beam spot image and determines whether the beam spot is outside a specified quality range as defined by the pass/fail conditions. In such an embodiment, QA evaluation module 520 analyzes the beam spot to determine one or more beam spot quality metrics, such as a beam spot area, a beam spot elongation, a beam spot power per unit area, and/or a beam spot center point offset from an ideal center point location. QA evaluation module 520 then compares the values determined for the one or more beam spot quality metrics to corresponding values included in standard P/F conditions 502 and/or user provided P/F conditions 503. In other examples, QA evaluation module 520 determines measured values for beam energy, beam profile, and the like, and compares the measured values to corresponding values included in standard P/F conditions 502 and/or user provided P/F conditions 503.

In some embodiments, QA evaluation module 520 can be implemented in control system 500 for the TDS as part of TDS controller 510. In embodiment illustrated in FIG. 5, QA evaluation module 520 is implemented as a separate software module that runs in control system 500.

QA application 550 runs locally on a TDS, for example as part of control system 500. In the embodiment illustrated in FIG. 5, QA application 550 includes a template generator 560 and a beam plan generator 570. Template generator 560 generates a beam plan template 561 for performing a particular QA test on the TDS associated with control system 500. As shown, template generator 560 generates each beam plan template 561 based on a QA testing guideline 504 for a particular QA test, system-specific capabilities 505, and, in some embodiments, user-provided P/F conditions 503. Generally, each beam plan template 561 includes specific operations to be performed by the TDS to perform a particular QA test. In some embodiments, such operations may include one or more mechanical operations, such as rotation of a gantry to a specified position and/or at a specified rotational velocity, positioning of a treatment couch at a specified position, and the like. Alternatively or additionally, in some embodiments, such operations may include one or more treatment- and/or imaging-beam operations. Each beam plan template 561 includes a set of predefined motions and operations that are executed by the TDS in order to perform that particular QA test.

It is noted that, in contrast to a beam plan template 561 for a particular QA test, a QA beam plan 571 for that particular QA test includes the specific machine-readable commands for controlling the TDS to perform the predefined motions and operations of a particular beam plan template 561. It is further noted that, because template generator 560 runs locally as part of control system 500, beam plan templates 561 are stored locally within the TDS associated with control system 500. As a result, when the QA test associated with beam plan template 561 is performed on the TDS, beam plan templates 561 are not imported from a separate system, such as a treatment planning system.

QA testing guideline 504 for a particular QA test can include guidelines published by the AAPM, the manufacturer of the TDS, and/or the like. Such guidelines provide specific information for various QA tests. For example, for a particular QA test, QA testing guideline 504 can provide values for beam parameters, positions for a gantry and/or treatment couch, collimator angle, field size, imaging settings, and the like.

System-specific capabilities 505 includes information that is related to the specific TDS to be performing a QA test. In some embodiments, system-specific capabilities 505 can include specific configuration information for the TDS to be performing the QA test, such as specific hardware features installed on or included in the TDS (e.g., imager type, imaging source type, treatment beam source type, collimator type, and the like). Alternatively or additionally, in some embodiments, system-specific capabilities 505 can include modified settings of the TDS, such as axis limits that have been established for the TDS to prevent, for example, couch collisions and the like. Alternatively or additionally, in some embodiments, system-specific capabilities 505 can include information indicating that certain features of the TDS are not currently licensed, and therefore are not available to the user. For example, when an appropriate license has been purchased by a user for a particular capability (e.g., high gantry speed), software enabling that capability is unlocked, and the availability of this capability is included in system-specific capabilities 505. As a result, a QA test that includes that particular capability can be made available to the user via the generation of an appropriate beam plan template 561 by template generator 560.

Beam plan generator 570 generates a QA beam plan 571 for performing a particular QA test on the TDS. In some embodiments, beam plan generator 570 generates the QA beam plan 571 based on a specific beam plan template 561 and on information included in system-specific capabilities 505. In some embodiments, beam plan generator 570 generates a QA beam plan 571 using techniques and algorithms employed by conventional treatment planning systems. However, because beam plan generator 570 runs locally as part of control system 500, QA beam plans 571 are stored locally within the TDS associated with control system 500. As a result, when the QA test associated with QA beam plan 571 is performed on the TDS, QA beam plans 571 are not imported from a separate system, such as a treatment planning system.

### Generating a Beam Plan Template Locally on TDS

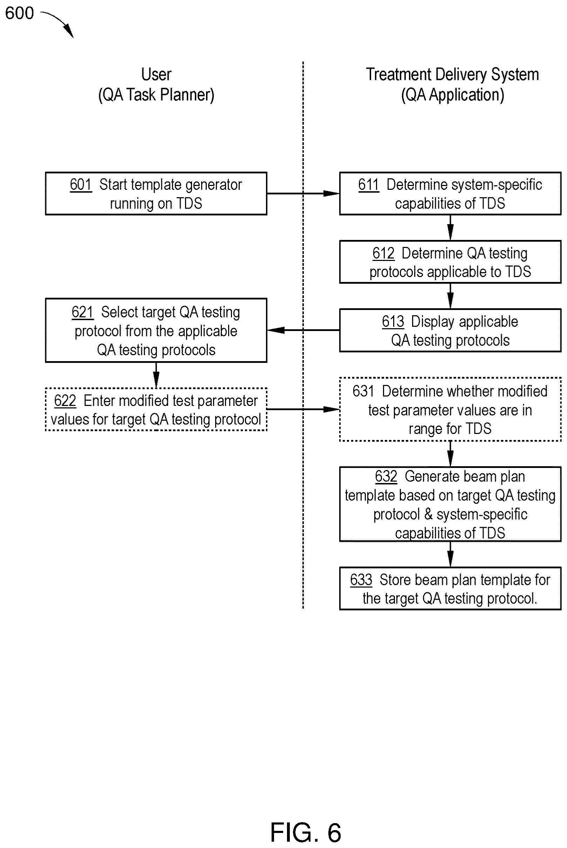

FIG. 6 is a flowchart illustrating the steps performed by QA application 550 and a user interacting with QA application 500 as part of a QA task planning process 600, according to various embodiments. QA task planning process 600 may include one or more operations, functions, or actions as illustrated by one or more of blocks 601 - 633. Although the blocks are illustrated in a specific order, these blocks may be performed in parallel, and/or in a different order than those described herein. Also, the various blocks may be combined into fewer blocks, divided into additional blocks, and/or eliminated based upon a specific implementation. Although the method is described in conjunction with control system 500 of FIG. 5, persons skilled in the art will understand that within the scope of the present disclosure any suitably configured system can perform QA task planning process 600.

In QA task planning process 600, steps are performed in sequence by a user, such as a QA task planner, and the TDS associated with control system 500. In some embodiments, the QA task planner can be a medical physicist or other technical expert tasked with the generation of a QA beam plan that enables the performance of a particular QA test by the TDS.

In some embodiments, QA task planning process 600 is performed to generate a beam plan template 561 so that a particular QA test can be subsequently performed on the TDS. In some embodiments, based on beam plan template 561, the particular QA test can be performed as part of initial acceptance testing of the TDS and/or the commissioning of the TDS for clinical use. Alternatively or additionally, in some embodiments, based on beam plan template 561, the particular QA test can be performed periodically (e.g., daily, weekly, monthly) and/or after significant repair, intervention, or adjustment is performed on the TDS or in response to an indication of a change in performance one or more subsystems of the TDS.

QA task planning process 600 begins at step 601, where the user starts QA application 550. For example, in some embodiments, the user logs on to the TDS and provides an input that selects the QA application from a suitable menu.

In step 611, in response to receiving the user input of step 601, QA application 550 determines system-specific capabilities 505. In some embodiments, QA application determines specific hardware features that are included in the TDS and/or software capabilities that are available for use. In some embodiments, in step 611, QA application 550 reads or otherwise determines what options are licensed on the TDS. In such embodiments, unlicensed options that the TDS is capable of using are not included in the system-specific capabilities 505 determined in step 611.

In step 612, QA application 550 determines the QA testing protocols applicable to the TDS. In some embodiments, QA application 550 determines the available QA testing protocols based on QA testing guidelines 504 and, in some embodiments, on one or more of the system-specific capabilities 505 determined in step 611. For example, when QA application 550 determines the TDS is equipped with an X-ray imaging system that acquires cone-beam computed tomography (CBCT) images, the testing protocols determined to be applicable to the TDS include one or more QA testing protocols for CBCT imaging. In another example, when QA application 550 determines the TDS is equipped with a treatment couch capable of rotation about an isocenter of the TDS, the testing protocols determined to be applicable to the TDS include one or more QA testing protocols for checking rotational alignment of the treatment couch.

In step 613, QA application 550 displays the testing protocols that are available on the TDS to the user, for example via remote control console 111 or remote control console 310. According to various embodiments, each available testing protocol can be implemented as a QA test on the TDS via a QA beam plan 571 that is generated based on a suitable beam plan template 561.

In step 621, the user selects a target QA testing protocol from the available QA testing protocols provided by QA application 550. In optional step 622, the user further enters one or more test parameter values and/or user provided P/F conditions 503 for the target QA testing protocol. Thus, in such embodiments, the user can modify the QA testing protocol provided by QA application 550 with the test parameter values and/or user provided P/F conditions 503 entered in step 622. In such embodiments, template generator 560 is configured to generate a beam plan template 561 that is also based on the one or more test parameter values and/or the user provided P/F conditions 503 entered in step 622. For example, in some instances, a particular user may employ the TDS with a gantry at a specific angle that is not included in the target QA testing protocol that is initially provided by QA application. In such instances, the particular user can modify the target QA testing protocol to include the user-specific gantry angle and/or to eliminate other gantry angles, so that the TDS undergoes QA testing that is tailored to the use of that specific TDS.

In optional step 631, which is performed in response to optional step 621, QA application 550 determines whether the modified test parameter values entered in optional step 621 are in-range for the TDS. In this way, the limited inputs by the user in QA task planning process 600 are checked for errors.

In step 632, QA application 550 generates a beam plan template 561 based on the target QA testing protocol selected in step 621 and on one or more of the system-specific capabilities 505 determined in step 611. In some embodiments, the beam plan template 561 generated by QA application 550 is further based on user-provided P/F conditions 503 and/or other test parameter values entered in optional step 622.

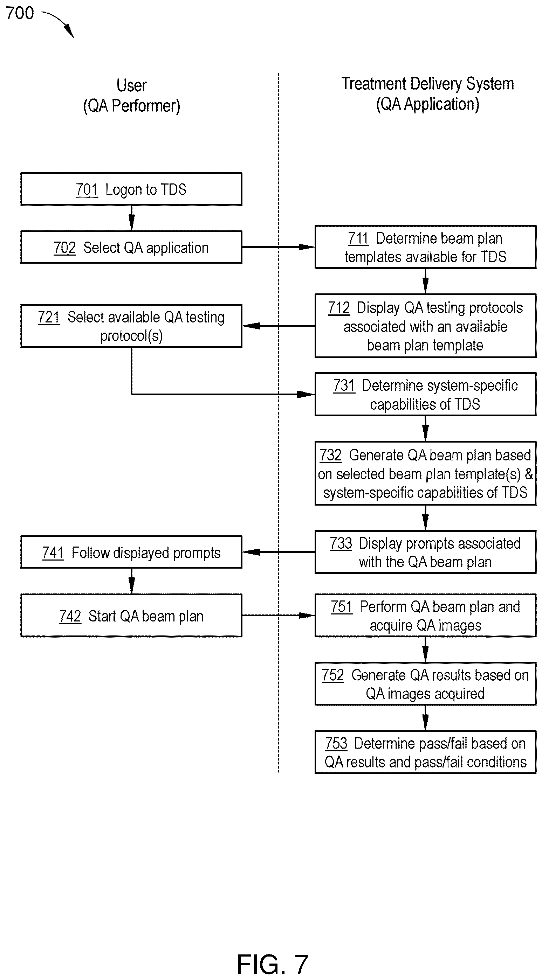

In step 633, QA application 550 stores the beam plan template 561 generated for the target QA testing protocol. For example, in some embodiments, QA application 550 stores the beam plan template 561 locally on the TDS. As a result, a QA test that conforms to the target QA testing protocol can be performed on the TDS without the importing of a beam plan from a separate treatment planning system. Furthermore, the QA test can be performed on the TDS without the logistical issues associated with scheduling a treatment plan on the TDS in advance. The performance of such a QA test is described below in conjunction with FIG. 7.

### Generating a QA Beam Plan Locally on TDS

FIG. 7 is a flowchart illustrating the steps performed by QA application 550 and a user interacting with QA application 500 as part of a QA process 700, according to various embodiments. QA task 700 may include one or more operations, functions, or actions as illustrated by one or more of blocks 701 - 753. Although the blocks are illustrated in a specific order, these blocks may be performed in parallel, and/or in a different order than those described herein. Also, the various blocks may be combined into fewer blocks, divided into additional blocks, and/or eliminated based upon a specific implementation. Although the method is described in conjunction with control system 500 of FIG. 5, persons skilled in the art will understand that within the scope of the present disclosure any suitably configured system can perform QA process 700.

In QA process 700, steps are performed in sequence by a user, such as a QA performer, and the TDS associated with control system 500. In some embodiments, the QA performer can be a radiation therapist, a medical physicist, or other user of the TDS responsible for daily, weekly, or monthly QA testing of the TDS.

In some embodiments, QA process 700 is performed to generate a beam plan 571 so that a particular QA test or series of multiple QA tests can be subsequently performed on the TDS. In some embodiments, the particular QA test (or series of QA tests) can be performed as part of initial acceptance testing of the TDS and/or the commissioning of the TDS for clinical use. Alternatively or additionally, in some embodiments, based on beam plan 571, the particular QA test (or series of QA tests) can be performed periodically (e.g., daily, weekly, monthly) and/or after significant repair, intervention, or adjustment is performed on the TDS or in response to an indication of a change in performance one or more subsystems of the TDS.

QA process 700 begins at step 701, where the user starts QA application 550. For example, in some embodiments, the user logs on to the TDS. In step 702, the user provides an input that selects the QA application from a suitable menu.

In step 711, in response to receiving the user input of step 702, QA application 550 determines the beam plan templates 561 that have been generated for the TDS or are otherwise available. As shown in FIG. 5, in some embodiments, beam plan templates 561 can be stored locally on the TDS. In step 712, QA application 550 displays QA testing protocols that are associated with an available beam plan template 561.

In step 721, the user selects a displayed QA testing protocol that corresponds to the QA test to be performed in QA process 700. For example, in some embodiments, the user provides an input to QA application 550 indicating selection of a QA testing protocol that is associated with the QA beam plan template corresponding to the QA test to be performed in QA process 700. In some embodiments, multiple displayed QA testing protocols can be selected in step 721 by the user, indicating that a beam plan 571 is to be generated that implements a series of multiple QA tests.

In step 731, QA application 550 determines one or more system-specific capabilities of the TDS. Specifically, QA application 550 determines system-specific capabilities of the TDS that can affect the execution of the QA beam plan 571 based on beam plan template 561. In this way, the effect of updated settings, hardware features, and/or software features of the TDS is captured without inputs provided by the user. In step 732, QA application generates a QA beam plan 571 based on the beam plan template (or templates) 561 selected in step 721 and on the system-specific capabilities of the TDS determined in step 731. In step 733, QA application 550 displays one or more prompts associated with QA beam plan 571, such as placement of a phantom, positioning of a treatment couch and/or gantry, and the like.

In step 741, the user responds to the prompts displayed in step 733. For example, in some embodiments, the user sets up a suitable phantom on a treatment couch of the TDS and then inputs an acknowledgment that the phantom has been positioned. In step 742, once all prompts have been responded to, the user starts QA beam plan 571. For example, in some embodiments, the user depresses a "beam-on" button or selects a "beam-on" icon on a display screen of the TDS.

In step 751, the TDS performs QA beam plan 571. As noted above, in some instances, QA beam plan 571 can cause a series of multiple QA tests to be performed on the TDS. In step 752, the TDS generates QA results, such as QA output 511. In some embodiments, the QA results can include kV images and/or MV images. Alternatively or additionally, in some embodiments, the QA results can include other measurements or data generated during execution of QA beam plan 571. In step 753, QA application determines pass/fail of the TDS based on the QA results and certain pass/fail conditions, such as standard P/F conditions 502 and/or user provided P/F conditions 503.

### Example Computing Device

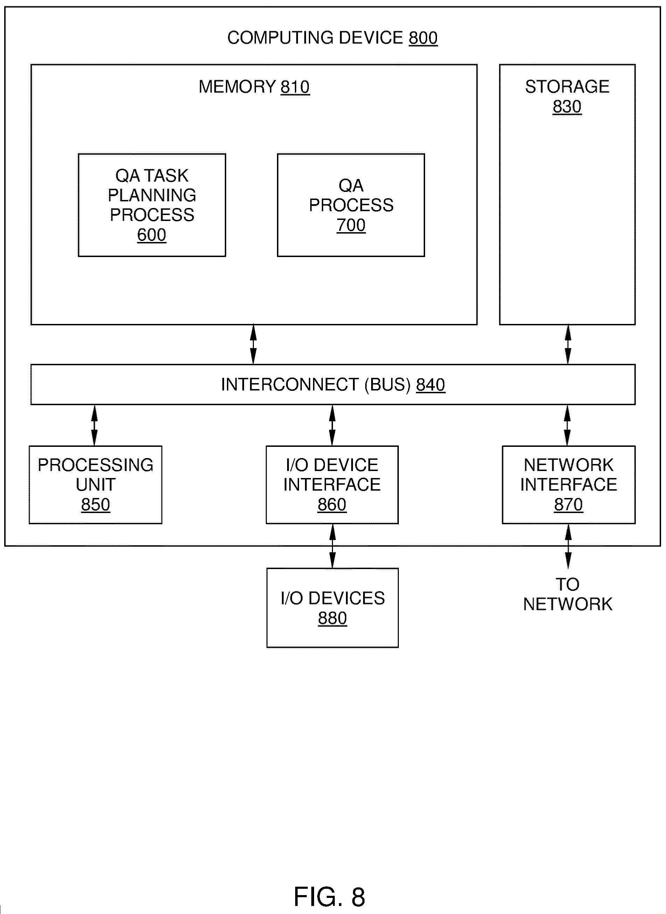

FIG. 8 is an illustration of computing device 800 configured to perform various embodiments of the present disclosure. For example, in some embodiments, computing device 800 can be implemented as image acquisition and treatment control computer 109 of RT system 100 or image acquisition and treatment control computer 306 of RT system 300. Computing device 800 may be a desktop computer, a laptop computer, a smart phone, or any other type of computing device suitable for practicing one or more embodiments of the present disclosure. In operation, computing device 800 is configured to execute instructions associated with QA task planning process 600 and/or QA process 700, as described herein. It is noted that the computing device described herein is illustrative and that any other technically feasible configurations fall within the scope of the present disclosure.

As shown, computing device 800 includes, without limitation, an interconnect (bus) 840 that connects a processing unit 850, an input/output (I/O) device interface 860 coupled to input/output (I/O) devices 880, memory 810, a storage 830, and a network interface 870. Processing unit 850 may be any suitable processor implemented as a central processing unit (CPU), a graphics processing unit (GPU), an application-specific integrated circuit (ASIC), a field programmable gate array (FPGA), any other type of processing unit, or a combination of different processing units, such as a CPU configured to operate in conjunction with a GPU or digital signal processor (DSP). In general, processing unit 850 may be any technically feasible hardware unit capable of processing data and/or executing software applications, including QA task planning process 600 and/or QA process 700.

I/O devices 880 may include devices capable of providing input, such as a keyboard, a mouse, a touch-sensitive screen, and so forth, as well as devices capable of providing output, such as a display device and the like. Additionally, I/O devices 880 may include devices capable of both receiving input and providing output, such as a touchscreen, a universal serial bus (USB) port, and so forth. I/O devices 880 may be configured to receive various types of input from an end-user of computing device 800, and to also provide various types of output to the end-user of computing device 800, such as displayed digital images or digital videos. In some embodiments, one or more of I/O devices 880 are configured to couple computing device 800 to a network.

Memory 810 may include a random access memory (RAM) module, a flash memory unit, or any other type of memory unit or combination thereof. Processing unit 850, I/O device interface 860, and network interface 870 are configured to read data from and write data to memory 810. Memory 810 includes various software programs that can be executed by processor 850 and application data associated with said software programs, including QA task planning process 600 and/or QA process 700.

### Example Computer Program Product

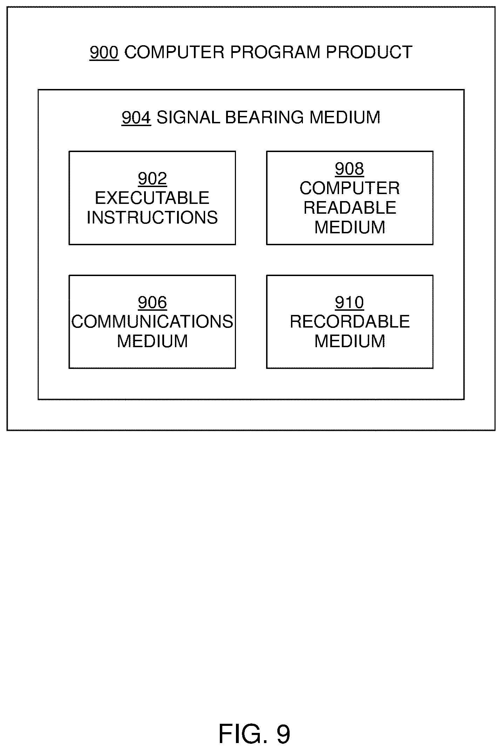

FIG. 9 is a block diagram of an illustrative embodiment of a computer program product 900 for implementing a method for performing a QA test on a treatment delivery system, according to various embodiments. Computer program product 900 may include a signal bearing medium 904. Signal bearing medium 904 may include one or more sets of executable instructions 902 that, when executed by, for example, a processor of a computing device, may provide at least the functionality described above with respect to FIGS. 1 - 8.

In some implementations, signal bearing medium 904 may encompass a non-transitory computer readable medium 908, such as, but not limited to, a hard disk drive, a Compact Disc (CD), a Digital Video Disk (DVD), a digital tape, memory, etc. In some implementations, signal bearing medium 904 may encompass a recordable medium 910, such as, but not limited to, memory, read/write (R/W) CDs, R/W DVDs, etc. In some implementations, signal bearing medium 904 may encompass a communications medium 906, such as, but not limited to, a digital and/or an analog communication medium (e.g., a fiber optic cable, a waveguide, a wired communications link, a wireless communication link, etc.). Computer program product 900 may be recorded on non-transitory computer readable medium 908 or another similar recordable medium 910.

The descriptions of the various embodiments have been presented for purposes of illustration, but are not intended to be exhaustive or limited to the embodiments disclosed. Many modifications and variations will be apparent to those of ordinary skill in the art without departing from the scope and spirit of the described embodiments.

Aspects of the present embodiments may be embodied as a system, method or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

While various aspects and embodiments have been disclosed herein, other aspects and embodiments will be apparent to those skilled in the art without departing from the scope and spirit of the described embodiments. The various aspects and embodiments disclosed herein are for purposes of illustration and are not intended to be limiting, with the true scope and spirit being indicated by the following claims.

## Claims

1. A computer-implemented method of performing a quality assurance, QA, test on a treatment delivery system, the method comprising:
with one or more processors of the treatment delivery system, generating a beam plan template for performing the QA test on the treatment delivery system, wherein the beam plan template is based on a testing guideline for the QA test;
in response to a first user input, determining, with the one or more processors, one or more system-specific capabilities of the treatment delivery system at the time of receipt of the first user input;
generating, with the one or more processors, a QA beam plan for performing the QA test on the treatment delivery system, wherein the QA beam plan is based on the beam plan template and the one or more system-specific capabilities of the treatment delivery system; and
causing, with the one or more processors, the treatment delivery system to perform the QA beam plan.

2. The computer-implemented method of claim 1, wherein the beam plan template includes specific operations to be performed by the treatment delivery system to perform the QA test.

3. The computer-implemented method of claim 1 or 2, wherein the testing guideline for the QA test comprises a published guideline for radiation therapy systems.

4. A treatment delivery system, comprising:
a radiation source mounted on a gantry that rotationally positions the radiation source about an isocenter of the treatment delivery system; and
a controller configured to perform the steps of:
generating a beam plan template for performing a quality assurance, QA, test on the treatment delivery system, wherein the beam plan template is based on a testing guideline for the QA test;
in response to a first user input, determining one or more system-specific capabilities of the treatment delivery system at the time of receipt of the first user input;
generating a QA beam plan for performing the QA test on the treatment delivery system, wherein the QA beam plan is based on the beam plan template and the one or more system-specific capabilities of the treatment delivery system; and
causing the treatment delivery system to perform the QA beam plan.

5. The computer-implemented method of claim 1, 2 or 3 or treatment delivery system of claim 4, wherein the beam plan template is further based on one or more physical features of the treatment delivery system.

6. The computer-implemented method or treatment delivery system of claim 5, wherein the one or more physical features of the treatment delivery system include at least one of an imager type, an imaging source type, a treatment beam source type, a collimator type, or a safety feature of the treatment delivery system.

7. The computer-implemented method of any one of claims 1, 2, 3, 5 or 6, or treatment delivery system of claim 4, 5 or 6, further comprising, in response to the first user input, determining the one or more physical features of the treatment delivery system.

8. The computer-implemented method of any one of claims 1, 2, 3, 5, 6 or 7, or treatment delivery system of claim any one of claims 4 to 7, wherein the one or more processors generate the QA beam plan in response to a second user input.

9. The computer-implemented method of any one of claims 1, 2, 3, 5, 6, 7 or 8, or treatment delivery system of claim any one of claims 4 to 8, wherein the first user input comprises an input selecting the beam plan template for the QA test.

10. The computer-implemented method or treatment delivery system of claim 9, wherein the QA test is based on a state of the treatment delivery system at the time of receipt of the first user input.

11. A non-transitory computer-readable medium that includes a set of instructions which, in response to execution by one or more processors of a treatment delivery system, cause the one or more processors to perform a method of performing a quality assurance, QA, test on the treatment delivery system, the method comprising:
with the one or more processors, generating a beam plan template for performing the QA test on the treatment delivery system, wherein the beam plan template is based on a testing guideline for the QA test;
in response to a first user input, determining, with the one or more processors, one or more system-specific capabilities of the treatment delivery system at the time of receipt of the first user input;
generating, with the one or more processors, a QA beam plan for performing the QA test on the treatment delivery system, wherein the QA beam plan is based on the beam plan template and the one or more system-specific capabilities of the treatment delivery system; and
causing, with the one or more processors, the treatment delivery system to perform the QA beam plan.

12. The computer-implemented method, treatment delivery system or non-transitory computer-readable medium of claim of any one of claims 1 to 11, wherein the QA beam plan includes machine-readable commands for causing the treatment delivery system to perform the QA test.

13. The computer-implemented method, treatment delivery system or non-transitory computer-readable medium of claim 12, wherein the QA beam plan has a file format that includes human-readable text.

14. The computer-implemented method, treatment delivery system or non-transitory computer-readable medium of claim 12, wherein the QA beam plan comprises one of an Extensible Markup Language, XML, file, a JavaScript Object Notation, JSON, file, or a Digital Imaging and Communications in Medicine, DICOM, file.

15. The non-transitory computer-readable medium of any one of claims 11 to 14, including instructions which, in response to execution by one or more processors of the treatment delivery system, cause the one or more processors to perform the method of any one of claims 1 to 3 or 5 to 10.
